# EUROPEAN PATENT SPECIFICATION

(11) **EP 0 873 133 B1**
(45) Date of publication and mention of the grant of the patent: **06.03.2002**
(21) Application number: 96923154.7
(22) Date of filing: 02.07.1996
(51) Int. Cl.: A61K 38/13, A61K 9/127, A61K 9/107

(54) **BIOLOGICALLY ACTIVE COMPOSITION COMPRISING CYCLOSPORINE**
CYCLOSPORIN ENTHALTENDE, BIOLOGISCH AKTIVE ZUBEREITUNG
COMPOSITION BIOLOGIQUEMENT ACTIVE CONTENANT DE LA CYCLOSPORINE

(30) Priority: 06.07.1995 SE 9502472
(43) Date of publication of application: 28.10.1998
(73) Proprietor: CAMURUS AB, 213 75 Malmö (SE)
(72) Inventor: LARSSON, Kare, S-237 34 Bjärred (SE); LJUSBERG-WAHREN, Helena, S-236 33 Höllviken (SE)
(74) Representative: Larsson, Kjell
(86) International application number: SE9600893
(87) International publication number: WO9702042

(56) References cited:
- EP-A- 0 539 319
- WO-A-88/00059
- US-A- 4 388 307
- LIQUID LIPID-WATER PHASES, Volume 5, 1994, KARE LARSSON, "Lipids-Molecular Organization, Physical Functions and Technical Applications", page 1 - page 237.

## Description

### TECHNICAL FIELD

The present invention relates to a biologically active composition based on an L2-phase and more specifically to such a composition containing a specific class of biologically active substances, viz. cyclosporins. Thus, it has unexpectedly been found that considerable advantages are accomplished as concerns the oral uptake of said active substances compared to other similar active substances in L2-phases.

The invention also relates to a process for the preparation of said composition and to such a composition for use for oral administration.

### BACKGROUND OF THE INVENTION

L2-phases of the type to which the present invention relates have been disclosed in detail in European patent specification No. 0 314 689. Thus, the background of the use of L2-phases, especially for medical purposes, is described in detail in said patent specification, which means that information in these respects can be found in the same. However, the present invention is based on the unexpected discovery that the use of an L2-phase of the kind referred to gives a drastically improved oral uptake of a cyclosporin as the biologically acctive substance in such compositions. This is neither disclosed nor suggested in said patent specification.

Pharmaceutical compositions containing cyclosporin in glyceride-containing carriers are also previously known per se, viz. from US-A-4,388,307 and
EP-A2-0 539 319, but said carriers have compositions different from that of the specific carrier upon which the composition according to the present invention is based. Furthermore, it is essential to note that the known compositions are conventional L1-compositions, and not L2-compositions as in the present case, and that they have not been shown to possess the advantageous properties of the composition according to the present invention, especially the extremely reproducible and selective uptake in the small intestine, as compared to the uptake of other peptides which may cause immunological reactions.

### SUMMARY OF THE INVENTION

In other words the present invention is based on the same type of L2-phase that is disclosed in EP-A-0 314 689 but having a novel, specific class of biologically active substances therein, viz. cyclosporins.

Cyclosporins is a group of compounds which chemically are cyclic peptides, and many of them possess immunosuppressive activities. The cyclosporin which has beyond comparison become the most important one is cyclosporin A, which is in fact often the compound referred to when speaking about cyclosporin in general.

Cyclosporin A is a potent immunosuppressive agent, which has been disclosed per se in literature and which is a cyclic peptide consisting of 11 amino acids and having a molecular weight of approximately 1.2 kDa. Many of the amino acids present therein are of an uncommon structure and are strongly hydrophobic. The hydrophobicity of cyclosporin A is further enhanced by the fact that seven of said amino acids are N-methylated.

However, according to the present invention it has unexpectedly been found that the L2-phase implements a considerably improved uptake of cyclosporins through the intestinal wall, and in a very reproducible way, as compared to similar compounds, such as other peptides.

Thus, as far as we know, there are no data in the literature which suggest that the uptake of peptides in the intestinal tract might be enhanced by the incorporation thereof in an L2-phase of the kind referred to. However, in accordance with the invention it has been found that cyclosporin A solubilized in the L2-phase is taken up very efficiently in the small intestine, according to those tests in rat which are disclosed below. "Normally" peptides must not be taken up in the intestinal tract, since this might cause immunological reactions. We have also made comparisons with other peptides, e.g. DDAVP, but not found the high uptake that was observed for cyclosporin A.

More specifically, the present invention relates to a biologically active composition based on A) an L2-phase comprising (a) at least one monoglyceride of an unsaturated fatty acid having 16-22 carbon atoms or a vegetable or animal oil transformed into such a monoglyceride, (b) at least one triglyceride of an unsaturated fatty acid having 16-22 carbon atoms or a vegetable or animal oil containing such a triglyceride, and (c) at least one polar liquid selected from the group consisting of water, glycerol, ethylene glycol and propylene glycol, the weight ratio of monoglyceride to triglyceride being within the range of from 1:1 to 3:1, and B) a biologically active substance dissolved or dispersed in said L2-phase, the characterizing feature of the composition being that said biologically active substance is a cyclosporin.

The composition according to the invention can contain any cyclosporin, but the preferred cyclosporin according to the present invention is cyclosporin A. Another interesting cyclosporin for use in the invention is cyclosporin G. Further examples of cyclosporins in connection with the invention are cyclosporin C, dihydrocyclosporin C, cyclosporin D, dihydrocyclosporin D and isocyclosporin D.

Another preferable embodiment of the composition claimed is represented by the case where the composition is adapted as a pharmaceutical composition and especially for oral administration, as said route of administration has been shown to give an unexpectedly high uptake of the active ingredient, which could not be theoretically foreseen.

Furthermore, another interesting observation in connection with the invention is that when varying the concentration of the cyclosporin in the L2-phase an uptake is achieved which is not proportional to the concentration. In other words especially advantageous results are achieved at increasing or higher concentrations.

Generally the cyclosporin can be utilized in such a low concentration as from 0.1% by weight up to the saturation concentration, based on the weight of the total composition, but in practice the lower limit typically is at least 1% by weight or at least 2% by weight, a preferable range being represented by 2-20% by weight, more preferably 5-20% by weight and most preferably 8-15% by weight.

Expressed in another way a specially preferable embodiment of the invention could be said to be represented by at least 8% by weight and up to the saturation concentration, based on the fact that the uptake of the active ingredient has been found to be higher at higher concentrations, i.e. the increase is higher than proportional to the concentration.

As was mentioned above the L2-phase per se has been closely described in EP-A-0 314 689, which means that detailed information thereabout can be found in said document. However, some advantages of the L2-phase and preferable embodiments of the invention can be summarized as follows.

The L2-phase is advantageous inter alia in that it is thermodynamically stable and therefore has no tendency to phase separate with time and in that it has distinct hydrophilic and hydrophobic domains, which enables the dissolution (solubilization) or dispersion of watersoluble as well as water-insoluble substances.

The distinct hydrophilic and hydrophobic domains represent an organized structure that puts restrictions on the diffusion of the added compound, a fact which can be advantageously utilized for controlled release thereof. Thus, the release rate of a bioactive substance is determined by the outer surface of the phase towards the surrounding medium and the proportions between hydrophilic and hydrophobic domains within the phase. As was mentioned above the L2-phase used in accordance with the present invention comprises or consists of at least one specific liquid monoglyceride, at least one specific liquid triglyceride and at least one polar liquid. Once these three ingredients of the system have been specified in each single case, the exact composition of the corresponding L2-phase can be found in the prior art, e.g. from a ternary phase diagram. An example of such a phase diagram, viz. for the system of sunflower oil monoglyceride/soybean oil/water at 40°C and 90°C, can be found in EP-A-0 314 689 referred to above.

Generally, the monoglyceride thus is a monoglyceride of an unsaturated fatty acid having 16-22 carbon atoms. However, often it is not necessary or even preferable to utilize said monoglyceride in the pure form. Rather it is preferably used in the form of a natural product essentially transformed into such a monoglyceride, such as a vegetable or animal oil essentially transformed into the appropriate monoglyceride. The measure of transforming an oil of the type referred to such that it comprises essentially the monoglyceride is a previously known process and need not be described more in detail herein. Thus, in principle the reaction is a re- or transesterification of the oil with an excess of glycerol.

In this context it could also be added that separation of saturated monoglyceride species by a fractionation of the total monoglyceride mixture can be performed in order to avoid a crystallization of the final L2-composition during storage thereof at refrigerator temperature. If for instance sunflower oil monoglycerides are used, the monopalmitin content can be drastically reduced by cooling the ethanol solution down to about 10-12°C and separation of the crystals at said low temperature.

According to a preferable embodiment of the invention the monoglyceride is a monoglyceride of an unsaturated fatty acid having 18 carbon atoms or a vegetable or animal oil transformed into the same. An especially preferable monoglyceride from this group is monoolein or monolinolein or the corresponding transformed vegetable or animal oil.

The triglyceride used is a triglyceride of at least one unsaturated fatty acid having 16-22 carbon atoms, but also in this case a natural product containing said triglyceride can replace the same, such as a vegetable or animal oil containing the desired triglyceride.

A preferable embodiment of the invention is represented by the case where the triglyceride is a triglyceride of at least one unsaturated fatty acid having 18 carbon atoms or a vegetable or animal oil containing the same, an especially preferable oil being soybean oil.

The polar liquid utilized in the claimed composition can be any of the liquids referred to and preferably mixtures thereof, which are preferable for the control or adjustment of the oral uptake of the biologically active material from the L2-phase. In other words different polar liquids or different proportions between polar liquids can be used to control or adjust the oral uptake of the active substance. A preferable mixture in this connection is a mixture of propylene glycol and glycerol. For such an adjustment of the oral uptake also common salt, i.e. sodium chloride, can be used.

As was mentioned above the exact composition of a specific L2-phase can be taken from a phase diagram while taking into consideration the desired oral uptake of the active compound, i.e. in this case a cyclosporin, which uptake or rate is determined by a person skilled in the art by simple animal tests. Generally, however, the weight ratio of monoglyceride to triglyceride is within the range of from 1:1 to 3:1, a preferable weight ratio being from 2:1 to 2.5:1, most preferably approximately 7:3.

The content of polar liquid is generally dictated by the maximum liquid content of the L2-phase region, which typically does not exceed 12-14% (weight/weight). A proper liquid content therefore is within the range of about 4-12, preferably about 5-10, % by weight.

As was mentioned above the composition according to the invention is especially interesting as a pharmaceutical composition and especially for oral administration. Therefore, a highly preferable embodiment of the invention is represented by a composition containing a pharmaceutically acceptable carrier adapted for oral administration.

According to another aspect of the invention there is provided a process for the preparation of the above-defined composition. Said process is characterized by forming a mixture of said monoglyceride and triglyceride in such amounts thereof that an L2-phase is formed when contacting said mixture with the polar liquid, and adding the cyclosporin to dissolve or disperse the same in said L2-phase, said addition being performed before, during or after the formation of said L2-phase.

Generally this means that the cyclosporin is added to the L2-phase after said phase has been formed, but it can also be added e.g. to the polar liquid before the L2-phase has been formed.

Before describing certain embodiments of the process according to the invention the following should be noted. The liquid aggregates of the L2-phase or the interphasial zone between the hydrophilic and hydrophobic regions of the phase provide the sites of oral uptake of the active substance dissolved in said phase. In the case of a very low solubility of the active substance in the phase, it can be dispersed within the same. The L2-phase has a very low interphasial tension towards an outside polar liquid phase and it is therefore easily emulsified in a polar liquid.

Since cyclosporins are a relatively sensitive group of substances, a preferable embodiment of the process claimed means that the cyclosporin is first dissolved in the polar liquid. Then the solution obtained is admixed with the monoglyceride-triglyceride mixture, the mixing operation preferably being performed by dropwisely adding the monoglyceride-triglyceride liquid to the cyclosporin solution.

This is the best way of maintaining the native peptide structure. If the drops are added to the cyclosporin solution with an interval around 1 sec, the L2-phase formed will swell to the limit of water swelling between each addition. In this way the cyclosporin will keep the water environment needed during the whole preparation process.

With reference to the process according to the invention it should also be added that preferable embodiments thereof correspond to those preferable embodiments which have been described above in connection with the composition. Therefore, such embodiments need not be repeated here.

Finally, the invention relates to the use of the above-mentioned L2-phase for the encapsulating of a cyclosporin for the manufacture of a preparation having an outstanding and controllable uptake profile for the active substance. Preferably said preparation is the preparation of an orally administrable preparation and especially for use as an immunosuppressive agent.

### EXAMPLES

Some embodiments of the invention will now be described more in detail by the following examples.

### EXAMPLE 1

### L2 for hard gelatin capsules

An L2-phase with 2, 6, 10 and 15% by weight of cyclosporin A was manufactured by dissolving the cyclosporin in a mixture containing 63% of glyceryl monooleate, 27% of glyceryl trioleate, 7.5% of propylene glycol and 2.5% of water. This cyclosporin formulation is a clear liquid having a low viscosity, which can be filled in hard gelatin capsules without any amended properties of said capsules after 10 days. In this context it should be noted that if the corresponding L2-phase having water as the only polar liquid is filled in capsules, said capsules will be tacky and deformed after 10 days.

### EXAMPLE 2

### L2-interindividual variation of the cyclosporin uptake in rats.

The interindividual variation of the uptake of cyclosporin from L2-phase containing 2, 6 and 10% from Example 1, after intragastric administration to rats, was studied and a summary thereof is presented in the table below. Said table presents the variance of the AUC*-values for 8 rats. The table indicates that the highest concentration is the best one, as it shows the lowest variance value.

| Cyclosporin concentration % | variance |
|---|---|
| 2% | 39% |
| 6% | 20% |
| 10% | 14% |
| * = Area under the curve | |

### EXAMPLE 3

### L2-self emulsifying cyclosporin preparation

This example shows that the uptake of cyclosporin from a lipid mixture with an L2-structure is independent of the addition of an emulsifying food stuff ingredient (casein). An L2-mixture of 2% of cyclosporin, 62% of glyceryl monooleate, 26% of glyceryl trioleate and 10% of water was prepared. This mixture was utilized either without further treatment or after dispersing the same in a 1.1% casein solution.

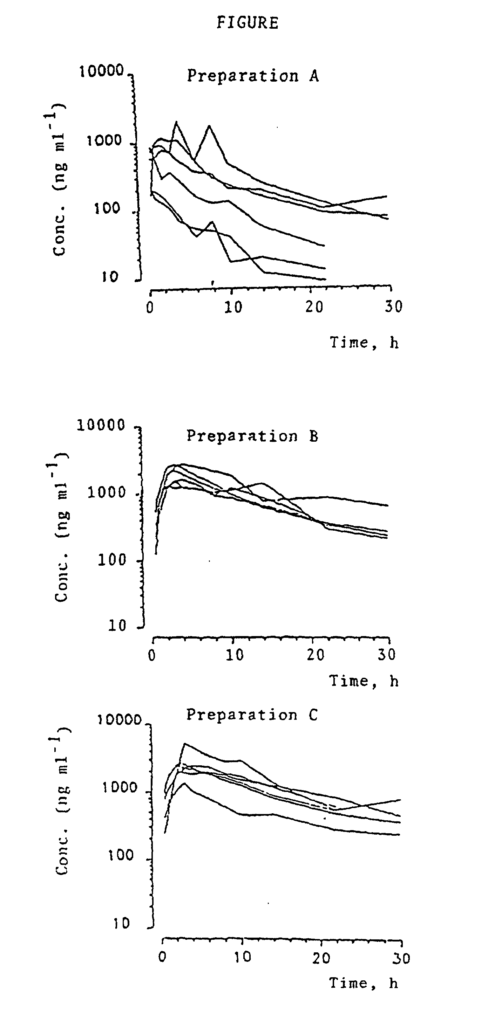

The uptakes of said preparations were studied in rats after intragastric administration, and the results are summarized in the enclosed Figure. Graph A shows the uptake of cyclosporin after intragastric administration of Sandimmun®. Graphs B and C show the uptake of cyclosporin after administration of an L2-mixture and an L2-phase dispersed in a casein solution, respectively.

According to the informtion we have Sandimmun® is Ciclosporin in a carrier of ethanol, maize oil and nonionic surfactant.

### EXAMPLE 4

### L2-cyclosporin preparation formed at body temperature

An L2-phase with 10% of cyclosporin was prepared by dissolving the cyclosporin at somewhat elevated temperature and stirring the same into 90% of L2-phase. The composition of the L2-phase was 63% of glyceryl monooleate, 27% of glyceryl trioleate, 7.5% of propylene glycol and 2.5% of glycerol.

The homogeneous preparation was subjected to freezing at -18°C, where the lipid mixture crystallized. At room temperature the mixture still is mainly crystalline and non-liquid, but at 37°C the mixture is an L2-phase of low viscosity.

### N.B.

Glyceryl monooleate and glyceryl trioleate referred to above are technical qualities thereof. The glyceryl monooleate was prepared from pine oil having a high content of oleic acid, while the glyceryl trioleate was prepared from sunflower oil.

## Claims

1. Biologically active composition based on A) an L2 phase comprising (a) at least one monoglyceride of an unsaturated fatty acid having 16-22 carbon atoms or a vegetable or animal oil transformed into such a monoglyceride, (b) at least one triglyceride of an unsaturated fatty acid having 16-22 carbon atoms or a vegetable or animal oil containing such a triglyceride, and (c) at least one polar liquid selected from the group consisting of water, glycerol, ethylene glycol and propylene glycol, the weight ratio of monoglyceride to triglyceride being within the range of from 1:1 to 3:1, and (B) a biologically active substance dissolved or dispersed in said L2 phase, **characterized in that** said biologically active substance is a cyclosporin.

2. A composition according to claim 1, **characterized in that** said cyclosporin is cyclosporin A.

3. A composition according to any one of the preceding claims, **characterized in that** said cyclosporin is present in an amount of from 1 % by weight up to the saturation concentration, based on the weight of the biologically active composition.

4. A composition according to claim 3, **characterized in that** said amount of cyclosporin is 2-20 % by weight, preferably 5-20 % by weight, and most preferably 8-15 % by weight.

5. A composition according to any one of the preceding claims, **characterized in that** said monoglyceride is a monoglyceride of an unsaturated fatty acid having 18 carbon atoms or a vegetable or animal oil transformed thereto.

6. A composition according to claim 5, **characterized in that** said monoglyceride is selected from the group consisting of monoolein and monolinolein or a vegetable or animal oil transformed thereto.

7. A composition according to any one of the preceding claims, **characterized in that** said triglyceride is a triglyceride of an unsaturated fatty acid having 18 carbon atoms or a vegetable or animal oil containing the same.

8. A composition according to claim 7, **characterized in that** said triglyceride is soy bean oil.

9. A composition according to any one of the preceding claims, **characterized in that** the weight ratio of monoglyceride to triglyceride is within the range of from 2:1 to 2.5:1, preferably around 7:3.

10. A composition according to any one of the preceding claims, **characterized in that** said polar liquid is a mixture of glycerol and propylene glycol.

11. A composition according to any one of the preceding claims, **characterized by** an amount of said polar liquid of about 4-12, preferably 5-10 % by weight.

12. A composition according to any one of the preceding claims for use as an orally administerable pharmaceutical composition, **characterized in that** it contains a pharmaceutically acceptable carrier adapted for oral administration.

13. A composition according to any one of the preceding claims, **characterized by** containing sodium chloride as an agent for modifying the oral uptake of the active substance.

14. A process for the manufacture of a biologically active composition according to any one of claims 1-13, **characterized by** forming a mixture of said monoglyceride and triglyceride in such amounts thereof that an L2 phase is formed when contacting said mixture with the polar liquid, and adding the cyclosporin to dissolve or disperse the same in said L2 phase, said addition being performed before, during or after the formation of said L2 phase.

15. A process according to claim 14, **characterized by** forming a mixture of the monoglyceride and the triglyceride, dissolving the cyclosporin in the polar liquid, preferably water, and adding, by drops, the monoglyceride-triglyceride mixture to the solution of cyclosporin in polar liquid.

16. Use of an L2 phase as defined in any one of claims 1-13 to encapsulate a cyclosporin for the manufacture of an orally administrable preparation for use as an immunosuppressive agent.

## Patentansprüche

1. Biologisch aktive Zusammensetzung auf der Grundlage von (A) einer L2-Phase, umfassend (a) mindestens ein Monoglycerid einer ungesättigten Fettsäure mit 16 bis 22 Kohlenstoffatomen oder ein in ein solches Monoglycerid umgewandeltes pflanzliches oder tierisches Öl, (b) mindestens ein Triglycerid einer ungesättigten Fettsäure mit 16 bis 22 Kohlenstoffatomen oder ein ein solches Triglycerid enthaltendes pflanzliches oder tierisches Öl und (c) mindestens eine polare Flüssigkeit ausgewählt aus der Gruppe bestehend aus Wasser, Glycerin, Ethylenglycol und Propylenglycol, wobei das Gewichtsverhältnis des Monoglycerids zu dem Triglycerid im Bereich von 1:1 bis 3:1 liegt, und (B) einer biologisch aktiven Substanz, die in der L2-Phase gelöst oder dispergiert ist, **dadurch gekennzeichnet, dass** die biologisch aktive Substanz ein Cyclosporin ist.

2. Zusammensetzung nach Anspruch 1, **dadurch gekennzeichnet, dass** das Cyclosporin Cyclosporin A ist.

3. Zusammensetzung nach einem der vorangehenden Ansprüche, **dadurch gekennzeichnet, dass** das Cyclosporin in einer Menge von 1 Gew.-% bis zur Sättigungskonzentration vorhanden ist, bezogen auf das Gewicht der biologisch aktiven Zusammensetzung.

4. Zusammensetzung nach Anspruch 3, **dadurch gekennzeichnet, dass** die Menge des Cyclosporins 2 bis 20 Gew.-%, vorzugsweise 5 bis 20 Gew.-% und am meisten bevorzugt 8 bis 15 Gew.-% beträgt.

5. Zusammensetzung nach einem der vorangehenden Ansprüche, **dadurch gekennzeichnet, dass** das Monoglycerid ein Monoglycerid einer ungesättigten Fettsäure mit 18 Kohlenstoffatomen oder ein dazu umgewandeltes pflanzliches oder tierisches Öl ist.

6. Zusammensetzung nach Anspruch 5, **dadurch gekennzeichnet, dass** das Monoglycerid ausgewählt ist aus der Gruppe bestehend aus Monoolein und Monolinolein oder ein dazu umgewandeltes pflanzliches oder tierisches Öl.

7. Zusammensetzung nach einem der vorangehenden Ansprüche, **dadurch gekennzeichnet, dass** das Triglycerid ein Triglycerid einer ungesättigten Fettsäure mit 18 Kohlenstoffatomen oder ein dieses Triglycerid enthaltendes pflanzliches oder tierisches Öl ist.

8. Zusammensetzung nach Anspruch 7, **dadurch gekennzeichnet, dass** das Triglycerid Sojabohnenöl ist.

9. Zusammensetzung nach einem der vorangehenden Ansprüche, **dadurch gekennzeichnet, dass** das Gewichtsverhältnis von Monoglycerid zu Triglycerid im Bereich von 2:1 bis 2,5:1, vorzugsweise etwa 7:3 liegt.

10. Zusammensetzung nach einem der vorangehenden Ansprüche, **dadurch gekennzeichnet, dass** die polare Flüssigkeit ein Gemisch aus Glycerin und Propylenglycol ist.

11. Zusammensetzung nach einem der vorangehenden Ansprüche, **dadurch gekennzeichnet, dass** die Menge der polaren Flüssigkeit etwa 4 bis 12, vorzugsweise 5 bis 10 Gew.-% beträgt.

12. Zusammensetzung nach einem der vorangehenden Ansprüche zur Verwendung als oral verabreichbares Arzneimittel, **dadurch gekennzeichnet, dass** es einen zur oralen Verabreichung angepassten pharmazeutisch verträglichen Träger enthält.

13. Zusammensetzung nach einem der vorangehenden Ansprüche, **dadurch gekennzeichnet, dass** sie Natriumchlorid als Mittel zur Modifizierung der oralen Aufnahme der aktiven Substanz enthält.

14. Verfahren zur Herstellung einer biologisch aktiven Zusammensetzung nach einem der Ansprüche 1 bis 13, **dadurch gekennzeichnet, dass** man ein Gemisch des Monoglycerids und Triglycerids in solchen Mengen erzeugt, dass eine L2-Phase erzeugt wird, wenn das Gemisch mit der polaren Flüssigkeit in Kontakt gebracht wird, und das Cyclosporin zum Lösen oder Dispergieren in der L2-Phase hinzufügt, wobei das Hinzufügen vor, während oder nach der Erzeugung der L2-Phase durchgeführt wird.

15. Verfahren nach Anspruch 14, **dadurch gekennzeichnet, dass** man ein Gemisch des Monoglycerids und Triglycerids erzeugt, das Cyclosporin in der polaren Flüssigkeit, vorzugsweise Wasser, löst und das Monoglycerid-Triglycerid-Gemisch zu der Cyclosporinlösung in der polaren Flüssigkeit tropfenweise hinzufügt.

16. Verwendung einer L2-Phase gemäß der Definition in einem der Ansprüche 1 bis 13 zur Einkapselung eines Cyclosporins für die Herstellung eines oral verabreichbaren Präparats zur Verwendung als immunsuppressives Mittel.

## Revendications

1. Composition biologiquement active basée sur A) une phase L2 comprenant (a) au moins un monoglycéride d'un acide gras insaturé ayant 16-22 atomes de carbone ou une huile végétale ou animale transformée en un tel monoglycéride, (b) au moins un triglycéride d'un acide gras insaturé ayant 16-22 atomes de carbone ou une huile végétale ou animale contenant un tel triglycéride, et (c) au moins un liquide polaire choisi dans le groupe consistant en l'eau, le glycérol, l'éthylèneglycol et le propylèneglycol, le rapport massique du monoglycéride au triglycéride étant dans le domaine de 1:1 à 3:1, et (B) une substance biologiquement active dissoute ou dispersée dans ladite phase L2, **caractérisée en ce que** ladite substance biologiquement active est une cyclosporine.

2. Composition selon la revendication 1, **caractérisée en ce que** ladite cyclosporine est la cyclosporine A.

3. Composition selon l'une quelconque des revendications précédentes, **caractérisée en ce que** ladite cyclosporine est présente en une quantité de 1% en masse jusqu'à la concentration de saturation, par rapport à la masse de la composition biologiquement active.

4. Composition selon la revendication 3, **caractérisée en ce que** ladite quantité de cyclosporine est de 2-20 % en masse, de préférence de 5-20 % en masse, et de préférence encore de 8-15 % en masse.

5. Composition selon l'une quelconque des revendications précédentes, **caractérisée en ce que** ledit monoglycéride est un monoglycéride d'un acide gras insaturé ayant 18 atomes de carbone ou une huile végétale ou animale transformée en celui-ci.

6. Composition selon la revendication 5, **caractérisée en ce que** ledit monoglycéride est choisi dans le groupe consistant en la monooléine et la monolinoléine ou une huile végétale ou animale transformée en celui-ci.

7. Composition selon l'une quelconque des revendications précédentes, **caractérisée en ce que** ledit triglycéride est un triglycéride d'un acide gras insaturé ayant 18 atomes de carbone ou une huile végétale ou animale contenant celui-ci.

8. Composition selon la revendication 7, **caractérisée en ce que** ledit triglycéride est l'huile de soja.

9. Composition selon l'une quelconque des revendications précédentes, **caractérisée en ce que** le rapport massique du monoglycéride au triglycéride est dans le domaine de 2:1 à 2,5:1, de préférence d'environ 7:3.

10. Composition selon l'une quelconque des revendications précédentes, **caractérisée en ce que** ledit liquide polaire est un mélange de glycérol et de propylèneglycol.

11. Composition selon l'une quelconque des revendications précédentes, **caractérisée par** une quantité dudit liquide polaire d'environ 4-12, de préférence 5-10 % en masse.

12. Composition selon l'une quelconque des revendications précédentes destinée à être utilisée comme composition pharmaceutique administrable oralement, **caractérisée en ce qu'**elle contient un support pharmaceutiquement acceptable adapté à l'administration orale.

13. Composition selon l'une quelconque des revendications précédentes, **caractérisée en ce qu'**elle contient du chlorure de sodium comme agent pour modifier l'absorption orale de la substance active.

14. Procédé de production d'une composition biologiquement active selon l'une quelconque des revendications 1 à 13, **caractérisé par** la formation d'un mélange dudit monoglycéride et dudit triglycéride en des quantités de ceux-ci telles qu'une phase L2 est formée lorsque ledit mélange est mis en contact avec le liquide polaire, et l'addition de la cyclosporine pour dissoudre ou disperser celle-ci dans ladite phase L2, ladite addition étant réalisée avant, pendant ou après la formation de ladite phase L2.

15. Procédé selon la revendication 14, **caractérisé par** la formation d'un mélange du monoglycéride et du triglycéride, la dissolution de la cyclosporine dans le liquide polaire, de préférence l'eau, et l'addition, goutte à goutte, du mélange monoglycéride-triglycéride à la solution de cyclosporine dans le liquide polaire.

16. Utilisation d'une phase L2 telle que définie dans l'une quelconque des revendications 1 à 13 pour encapsuler une cyclosporine pour la production d'une préparation administrable oralement destinée à être utilisée comme agent immunosuppresseur.
